# EUROPEAN PATENT SPECIFICATION

(11) **EP 1 377 336 B1**
(45) Date of publication and mention of the grant of the patent: **11.06.2008**
(21) Application number: 01926825.9
(22) Date of filing: 10.04.2001
(51) Int. Cl.: A61M 31/00, A61M 5/315, A61M 15/08, B05B 11/00

(54) **DRUG DELIVERY SYSTEM INCLUDING HOLDER AND DRUG CONTAINER**
ARZNEIVERABREICHUNGSSYSTEM MIT HALTER UND ARZNEIMITTELBEHÄLTER
SYSTEME D'ADMINISTRATION DE MEDICAMENTS COMPRENANT UN SUPPORT ET UN CONTENANT DE MEDICAMENTS

(43) Date of publication of application: 07.01.2004
(73) Proprietor: Becton, Dickinson and Company, Franklin Lakes, New Jersey 07417-1880 (US)
(72) Inventor: VEDRINE, Lionel, F-38400 St. Martin d'Heres (FR); JANSEN, Hubert, F-38560 Jarrie (FR); GRIMARD, Jean, Pierre, F-38450 Vif (FR); GUILLORY, Michel, F-38249 Meylan (FR)
(74) Representative: Selting, Günther
(86) International application number: PCT/US2001/011689
(87) International publication number: WO 2002/083230

(56) References cited:
- EP-A- 1 129 786
- WO-A-99/32185
- DE-A- 19 944 209
- US-A- 4 962 868
- US-A- 5 284 132
- US-A- 5 431 155
- US-A- 5 951 526

## Description

### FIELD OF THE INVENTION

The present invention generally relates to delivery systems for delivering substances such as drugs, vaccines and the like, and more specifically relates to a drug delivery system for preferably delivering such substances intranasally, i.e., through the nose, including a holder and a prefilled drug container such as a syringe. In addition, the present invention relates to a holder for use by one hand in which the travel of the stopper is controlled to insure the application of a minimum force before activation and to divide the substance to be delivered into at least two doses.

### BACKGROUND OF THE INVENTION

A number of medications may be effectively administered through the nasal passages. Devices have accordingly been developed for this purpose including either cartridges, such as those described in U.S. Patent Nos. 5,893,484 (Fuchs et al.), 5,813,570 (Fuchs et al.), 5,655,689 (Fuchs et al.), 5,511,698 (Solignac), 5,427,280 (Fuchs), 5,289,818 (Citterio et al.), 5,284,132 (Geier) and 5,171,219 (Fujioka et al.), or syringes, such as those described in U.S. Patent Nos. 5,601,077 (Imbert), 4,923,448 (Ennis, III), 4,767,416 (Wolf et al.) and 4,344,573 (De Felice).

The nasal syringes are usually of a more conventional construction such as that described in U.S. Patent No. 5,601,077 (Imbert) which includes a cylindrical barrel having a blunt tip portion for insertion into a nostril. A stopper is positioned within the barrel. A plunger extends from the end of the barrel opposite to the blunt tip. The plunger controls the position of the stopper within the barrel. A flange may be provided on one end of the plunger to facilitate its use. However, limitations remain, particularly with respect to insuring that sufficient force is applied to the plunger to obtain a therapeutic effective spray.

Nasal syringes are often supplied to users pre-filled with medication. Whether pre-filled or not, it may be desirable to administer selected, and usually equal volumes of medication to each nostril. U.S. Patent No. 4,962,868 (Borchard) discloses the use of a telescoping tube assembly which is designed for expelling the contents of a nasal syringe in two controlled doses. Also, U.S. Patent No. 5,601,077 (Imbert) discloses the use of a dose limited in the form of a c-shaped attachment on the plunger rod for limiting movement in the distal directed so that approximately half of the substance to be delivered remains in the syringe. To continue, the user simply removes the attachment from the plunger rod. In addition, U.S. Patent No. 5,951,526 (Korisch et al.) discloses a holder having an integral dose divider. However, it remains difficult for users to administer equal doses of medication to each nostril, especially with one hand.

A system for delivering a substance in at least two doses, corresponding to the first part of claim 1, is disclosed in DE 199 44 209 A1. This system comprises a drug container in the form of an ampoule comprising a stopper, a holder for guiding an actuating element comprising the ampoule. The actuating element is movable within the holder. The delivery of the medium contained in the ampoule is effected by a sequence of manual partial actuations of the actuating element in well defined partial doses. Between two partial actuations of the actuating element a switching actuation is provided.

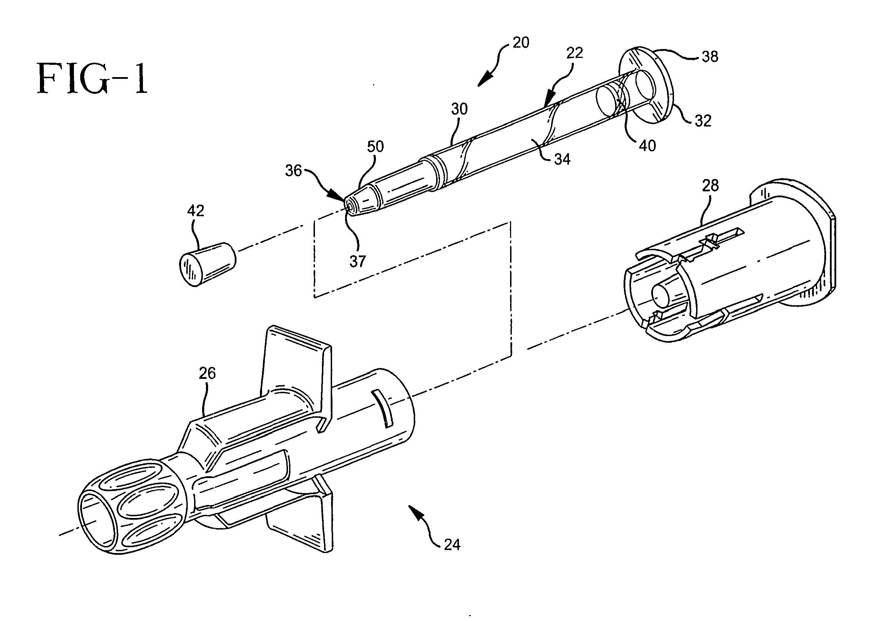

EP 1 129 786 A2 shall be considered as comprised in the state of the art according to Article 54 (3) EPC. This application describes a system for delivering at least one substance in at least two doses, which basically corresponds to Figs. 1-15.

Accordingly, there has been a need for a nasal drug delivery system which over comes the problems and limitations associated with the use of the prior devices for delivering a substance, especially intranasally easily with one hand, including delivery of a uniform spray which can be divided into at least two separate doses for delivery into each nostril. Also, there has been a need for a system which would permit the user to observe the substance in the system to determine, for example, if the system has been previously used.

### SUMMARY OF THE INVENTION

The system for delivering at least one substance in at least two doses of the present invention is defined by claim 1.

Accordingly, the system is characterized in that it comprises a pair of exterior ribs on the distal portion of the holder, wherein the slots in the proximal portion of the holder include at least one pair of slots situated thereof, with said pair of slots including a first slot and a second slot extending axially along the body of the proximal portion of the holder generally parallel to each other and dimensioned and situated to accommodate the ribs so that one of said ribs is insertable into each slot and able to travel along the slot upon activation of the system, wherein said distal portion and said proximal portion of said holder are attached to one another by means of flexible members and corresponding openings to avoid premature activation of the system.

Specifically, the invention is directed to an assembly such as a housing which allows the use of a conventional, pre-filled drug containers such as syringe while providing control of the dose to be administered. The assembly is particularly applicable to nasal syringes where it is often desirable to dispense medication in two equal doses.

The system of the present invention for delivering at least one substance in at least two doses includes a drug container including a barrel, a first end extending from the barrel, and a stopper slidably positioned within the barrel, a holder having a distal portion and a proximal portion, with the distal portion being assembled to the proximal portion, with the drug container secured therein, and means for controlling the delivery of a substance contained in the barrel of the drug container including a plurality of slots extending axially along at least one of the portions of the holder whereby upon activation of the system, the portions of the holder move towards one another upon the application of a minimum force and the stopper moves a preselected axial distance to expel at least a portion of the substance from the drug container.

In the preferred embodiment of the system, the first end of the drug container includes a spray nozzle for use in intranasally delivering the substance and the drug container is a syringe, and the distal portion of the holder acts as a plunger rod during activation of the system. In addition, the distal portion and the proximal portion of the holder each has a generally tubular interior configured to accommodate the drug container filled with a substance to be delivered and the proximal portion of the holder includes a closed end having a rod extending therefrom for engagement with the stopper of the drug container upon activation. Also, the preselected axial distance corresponds to about a dosage of the substance held in the drug container barrel desired to be administered in a first motion of the stopper, with the preselected axial distance preferably corresponding to about half the distance that the stopper is capable of moving within the barrel to administer about half of the substance held by the drug container.

Also, in the preferred system of the present invention, the distal portion includes a skirt extending from the distal portion, including the bridging portion, and the skirt covers the means for controlling the delivery of the substance. Also, the first end of the drug container includes a spray nozzle for use in intranasally delivering the substance and the drug container is a syringe, and a limiter is also associated with a first end of the distal portion, with the limiter limiting the depth of insertion of the spray nozzle into a nostril. Either the limiter or the spray nozzle is formed of a curved design to target specific areas in a nasal cavity. Further, the system includes a cap covering at least a first end of the distal portion, with the cap including a tamper evident means.

In addition, the preferred system of the present invention includes a means for securing the drug container in the distal portion of the holder with the distal portion having a first end through which the first end of the drug container can extend and a second, open end defining an opening of sufficient size for receiving the drug container. Specifically, the drug container is preferably a syringe having a rim extending from an open end thereof and the drug container securing means is situated adjacent the open end of the distal portion and includes at least one detent situated adjacent the open end and dimensioned so that the rim of the syringe may be securely retained in the distal portion by the detent. Also, a pair of flanges extend radially outwardly from the distal portion of the holder and attached thereto by a plurality of ribs, and the distal portion of the holder includes at least one window to permit visual inspect of the contents of the drug container located within the holder.

Also, the first slot is preferably open and is divided into at least two portions, and situated adjacent an open end of the first slot is a bridge extending across at least a portion of the slot, with the bridge being dimensioned so that when a rib comes in contact with the bridge and sufficient force is applied there against, the bridge will fracture to allow passage of the rib along the slot. In addition, a detent is situated adjacent the open end of the first portion of the first slot so that the rib can be clipped between the detent and the bridge prior to activation of the system, and the second portion of the first slot is at least slightly offset from the first portion of the first slot and towards the second slot, and the other rib travels along the second slot to provide structural stability and tracking, with the second slot including biasing means for biasing the rib in the first slot towards the second portion of the slot upon release of the force applied by a user. The biasing means is adapted to include a cut-away portion forming a deflectable arm having an inner wall associated with the second slot so that as the ribs travel along their respective slots, the one rib will deflect the flexible arm to cause the proximal portion of the holder to rotate relative to the distal portion about a central axis so that the rib situated in the first slot can come in contact with a second bridge so that upon sufficient force being applied, the bridge will fracture to allow passage of the rib along the second portion of the first slot.

The holder of the present invention according to Claim 22 includes a distal portion and a proximal portion, each configured to accommodate a drug container filled with a substance to be delivered, with the distal portion being able to be assembled to the proximal portion, and means for controlling the delivery of the substance including a plurality of slots extending axially along at least one of the portions of the holder whereby when the portions of the holder are moved towards one another upon the application of a minimum force, at least a portion of the substance can be expelled from the drug container.

In the preferred embodiment of the holder, a first end of the drug container includes a spray nozzle and the drug container is a syringe, and the distal portion and the proximal portion each has a generally tubular interior configured to accommodate the syringe and the proximal portion of the holder includes a closed end having a rod extending therefrom for engagement with the stopper of the syringe during activation.

The system of the present invention for the nasal delivery of at least one substance includes a syringe having a barrel, a first end extending from the barrel, the first end including a spray nozzle having an opening for dispensing the substance from the barrel, and at least one stopper slidably positioned within the barrel, a holder having a distal portion and a proximal portion, each configured to accommodate the syringe, with the distal portion being able to be assembled to the proximal portion, which acts as a plunger rod during activation of the system, and means for controlling the delivery of a substance including a plurality of slots extending axially along at least one of the portions of the holder whereby upon activation of the system, the portions of the holder move towards one another upon the application of a minimum force and the stopper moves a preselected axial distance to expel at least a portion of the substance from the syringe, with the preselected axial distance corresponding to about half the distance that the stopper is capable of moving within the barrel to administer about half of the substance contained by the syringe barrel.

In the system, a pair of flanges extend radially outwardly from the distal portion of the holder and are attached there along by a plurality of ribs, and the distal portion and the proximal portion each have a generally tubular configuration. In addition, the slots in the proximal portion of the holder include two corresponding sets situated on each side thereof, with each set including a first slot and a second slot extending axially along the body of the proximal portion of the holder generally parallel to each other and dimensioned and situated to accommodate the ribs so that the ribs are insertable into the slots and able to travel along the slots upon activation of the system, and the first slot is preferably open and is divided into at least two portions, and situated adjacent an open end of the first slot is a bridge extending across at least a portion of the slot, with the bridge being dimensioned so that when a rib comes in contact with the bridge and sufficient force is applied there against, the bridge will fracture or deform to allow passage of the rib along the slot. In addition, a detent is situated adjacent the open end of the first portion of the first slot so that the rib can be clipped between the detent and the bridge prior to activation of the system and the second portion of the first slot is at least slightly offset from the first portion of the first slot and towards the second slot. One of the ribs travels along each of the second slots to provide structural stability and tracking, with each second slot including a cut-away portion forming a deflectable arm for biasing the ribs in the first slots towards the second portions of the slots upon release of the force applied by a user so that as the ribs travel along their respective slots, the ribs traveling along the second slots will deflect the flexible arms to cause the proximal portion of the holder to rotate relative to the distal portion about a central axis so that the ribs situated in the first slots each come in contact with a second bridge so that upon sufficient force being applied, the bridges will each fracture or deform to allow passage of the ribs along the second portions of the first slots.

### BRIEF DESCRIPTION OF THE DRAWINGS

The various features, objects, benefits, and advantages of the present invention will become more apparent upon reading the following detailed description of the preferred embodiment along with the appended claims in conjunction with the drawings, wherein like reference numerals identify corresponding components, and:

Figure 1 is an exploded, perspective view of the holder and syringe of the drug delivery system not belonging to the present invention;

Figure 2 is a top perspective view of the assembled drug delivery system;

Figure 3 is a side view of the two portions of the holder prior to assembly;

Figure 4 is top view of the two portions of the holder prior to assembly;

Figures 5-9 are various side views of the holder showing the two portions assembled at various stages of activation to deliver doses of the substance contained therein;

Figures 10-12 are various top, sectional views of the syringe and holder at various stages during activation of the drug delivery system showing the position of the plunger within the syringe;

Figures 13 and 14 are schematic representations of the drug delivery system being used to deliver the substance to each of the nostrils of the user;

Figure 15 is a fragmentary, sectional view of an alternative embodiment of the drug delivery system illustrating securement of the syringe in the holder;

Figure 16 is top view of an alternative embodiment of the assembled drug delivery system of the present invention with two portions of the holder clipped together;

Figure 17 is top view of another alternative embodiment of the assembled drug delivery system of the present invention with two portions of the holder clipped together, with Figure 17A being a partial sectional side view of Figure 17;

Figure 18 is top view of yet another alternative embodiment of the assembled drug delivery system of the present invention with two portions of the holder clipped together, with Figure 18A being a partial sectional side view of Figure 18;

Figure 19 is a fragmentary perspective view of a limiter attached to the drug delivery system;

Figure 20 is a partial sectional top view of the proximal portion of the holder illustrating attachment of the limiter;

Figure 21 is a fragmentary perspective view of an alternative embodiment of the spray nozzle;

Figure 22 is top perspective view of a further embodiment of the assembled drug delivery system of the present invention with the proximal portion covered by a cap, which also holders the two portions of the holder together, with Figure 22A being a partial sectional side view of Figure 22; and

Figure 23 is top perspective view of the assembled drug delivery system illustrated in Figure 22 illustrating removal of the cap.

### DETAILED DESCRIPTION OF THE INVENTION

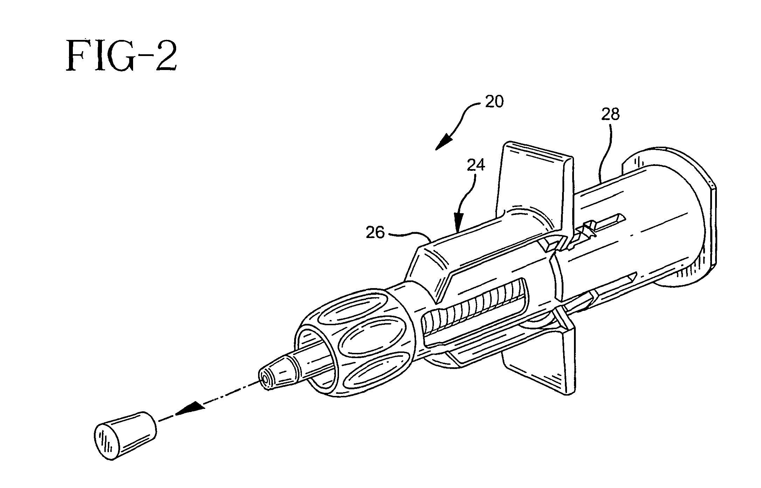

A drug delivery system of EP 1 124 786 A2 is illustrated in Figures 1-15, and generally includes the designation 20. Referring to Figures 1 and 2, the system 20 of the present invention includes a drug container such as preferably a syringe 22 and a holder 24. When used to delivery substances intranasally, the assembly insures that the substance to be delivered by the syringe is sprayed into the nostril and can be divided into at least two doses. It further limits the penetration of the syringe tip into the nostril and permits visual inspection of the substance contained in the system.

Referring to Figures 1 and 2, the holder 24 for the syringe is comprised of at least two portions, i.e., a distal portion 26 and a proximal portion 28, each have a generally tubular interior configuration to accommodate the syringe 22 therein. For the purposes of the description of the present invention, the term "distal" is meant to refer to the portion or end furthest from the person holding the system of the present invention and the term "proximal" is meant to refer to the portion or end closest to the person holding the system.

The particular syringe of the system is not essential to the present invention and may include, for example, when used to delivery substances intranasally, the nasal syringe disclosed in U.S. Patent No. 5,601,077 (Imbert). However, preferably, the syringe 22 includes an elongated cylindrical barrel 30 made of either glass or plastic having an open proximal end 32, a chamber 34 for retaining the substance to be delivered and a distal end or tip 36, with the chamber 34 extending from the proximal end to the distal end 36. The distal end includes an orifice 37 through which the substance can be expelled from the chamber. In the preferred embodiment, the proximal end 32 of the syringe includes a rim 38 extending therefrom. The syringe is preferably pre-filled with the substance such as a drug, vaccine or the like to be delivered prior to inserting the syringe into the holder 24.

A stopper 40 is slidably positioned in fluid-tight engagement inside the barrel 30. A sealing or tip cap 42 may be fitted over the tip 36 to prevent the loss of fluid through the orifice prior to use of the assembly.

A spray nozzle 50 extends outwardly from the tip portion of the barrel and includes a conduit therethrough in fluid communication with the orifice. The spray nozzle includes a distal end having a spray aperture in fluid communication with the conduit It will be apparent to one skilled in the art that there are numerous constructions that can be used to form the spray nozzle and that the arrangement described herein is exemplary of these many possibilities. Also, it is within the purview of this invention to include a valve-less nozzle. Accordingly, the preferred spray nozzle is taught in the prior art and commercially available such as for example from SOFAB of Paris, France and taught in SOFAB's French Patent No. 2,635,084.

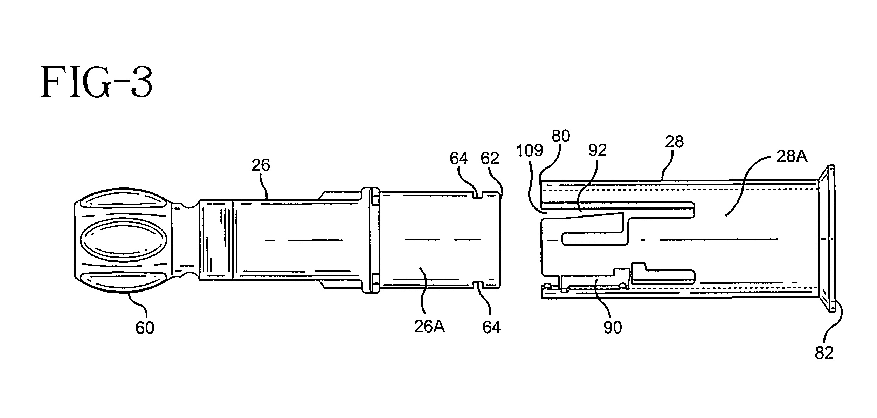

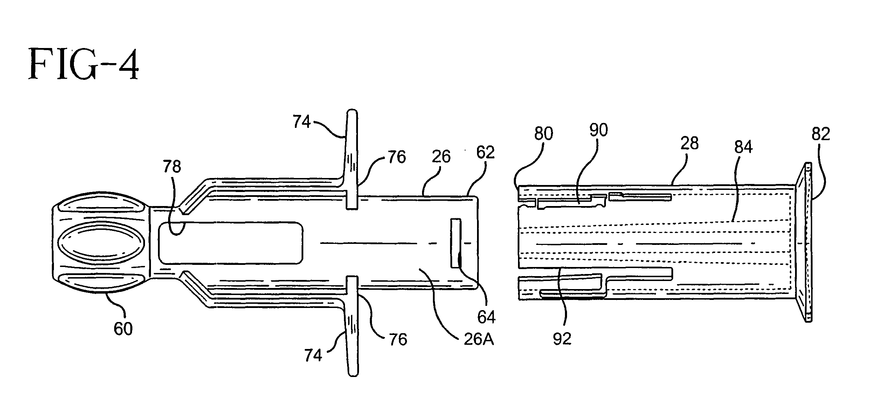

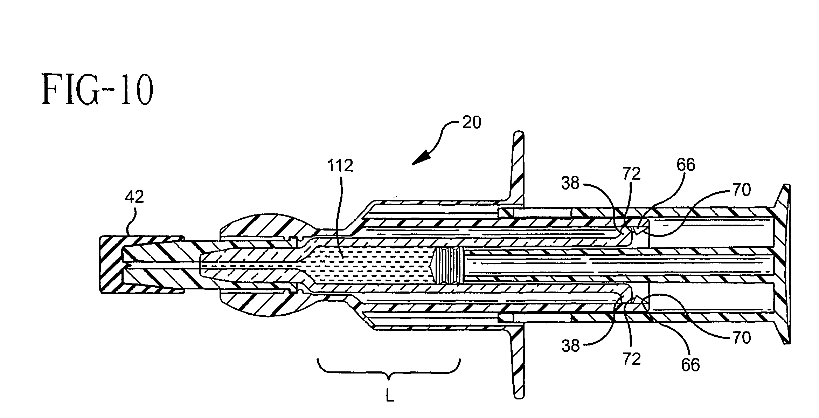

Referring to Figures 3 and 4, the two portions 26, 28 of the holder 24 are ergonomically shaped to facilitate handling and preferably have a generally tubular configuration to accommodate the syringe. In addition, the two portions 26, 28 are preferably made from a plastic material such as polypropylene. The distal portion 26 includes an elongated body 26A having a first, enlarged end 60 through which the tip 36, tip cap 42 and spray nozzle 50 can extend, and a second, opened end 62 defining an opening of sufficient size for receiving the syringe barrel, but which is preferably equal in size than the rim 38 through which the syringe may be inserted into the distal portion of the holder. In this way, preferably, the syringe is securely retained in the distal portion of the holder by a pair of grooves 64 and detents 66 situated proximally to the open end and dimensioned so that the rim of the syringe may extend therefrom. To facilitate insertion of the rim past the detents, as well as inhibit removal of the syringe from the holder, a projection to form the detent 66, as well as the groove, is situated adjacent each groove for retaining the syringe in the distal portion 26 of the holder 24 (Figures 10-12). Each detent 66 includes angled end face 70 and a shoulder portion 72 defined by the inner surface of the detent which allows the rim of the syringe to snap behind the detents and lock the rim of the syringe in place.

In addition, a pair of flanges 74 extending radially outwardly from the first end 60 towards the second open end 62 and are attached therealong by a plurality of ribs 76 (Figure 4). The front end 60 is larger in diameter than the diameter of an average adult nostril, and is blunt. While the embodiment shown in the drawings has a front end face which is rounded, it may alternatively be oval or any other shape desired, provided that the front end face is prevented from entry into the nostril.

One or more windows 78 are provided in the body 26A of the distal portion of the holder (Figure 4). The windows may be in the form of openings in the body, or transparent wall portions mounted to the body. The windows allow the user to view the syringe barrel located within the holder. The user can accordingly determine whether there is any substance present in the syringe, and whether the substance is suitable for administration. As some pharmaceutical products are frozen during storage, it may be important to determine whether the product within the syringe has thawed prior to administration.

The proximal portion 28 of the holder is designed to be assembled to the distal portion 26 and preferably acts as a plunger rod during activation of the system. In addition, the proximal portion includes a means for controlling delivery of the substance, as well as to divide the substance into at least two doses. Specifically, the proximal portion includes an end face defining an opening 80 of sufficient size for accommodating at least a portion of the body 26A of the distal portion 26 of the holder. In the preferred embodiment, the other end 82 is closed and includes an elongated rod 84 integrally formed in the proximal portion of the holder and extending from the closed end 82 towards the open end 80. The rod 84 is of a sufficient size and dimension to engage the stopper during activation of the system. However, it should be appreciated that a conventional plunger rod may be utilized with the syringe instead of having an integrally formed rod 84 in the holder.

Referring to Figures 3-9, the means utilized to control delivery of the substance includes a plurality of slots extending axially along the body of the proximal portion of the holder. The slots are preferably provided in two corresponding sets situated on each side thereof. Specifically, each set includes a first slot 90 and a second slot 92 extending axially along the body of the proximal portion of the holder generally parallel to each other. The slots are dimensioned and situated about the body 28A of the proximal portion 28 of the holder to accommodate the ribs 76 connecting the flanges 74 to the body 26A of the distal portion 26 so that the ribs are insertable into the slots and able to travel along the slots upon activation of the system.

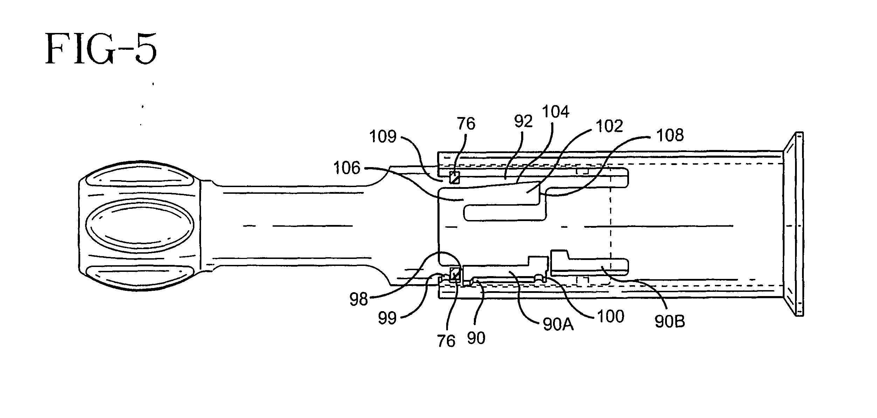

Referring to Figure 5, the first slot 90 is preferably open and is divided into at least two portions 90A, 90B when the system is to be used to deliver at least two doses of the substance, with each portion 90A, 90B having a length corresponding to the desired distance in which the stopper must travel to expel the substance from the chamber of the syringe barrel. Situated adjacent the open end of the slot 90 is a bridge 98 extending across at least a portion of the slot, and preferably bridging the entire gap of the slot. The bridge is dimensioned so that when a rib 76 comes in contact with the bridge and sufficient force is applied there against, the bridge will fracture and allow passage of the rib along the slot. In this way, the rib will travel along the slot until is comes in contact with a stop 100 situated at the end of the first portion 90A of the slot 90. The second portion 90B of the slot 90 is preferably slightly offset from the first portion 90A of the slot and towards the other slot 92.

A detent 99 is situated adjacent the open end 80 of the proximal portion of the holder along the slot 90 so that the rib can be clipped or snapped between the detent 99 and the bridge 98 prior to activation of the system.

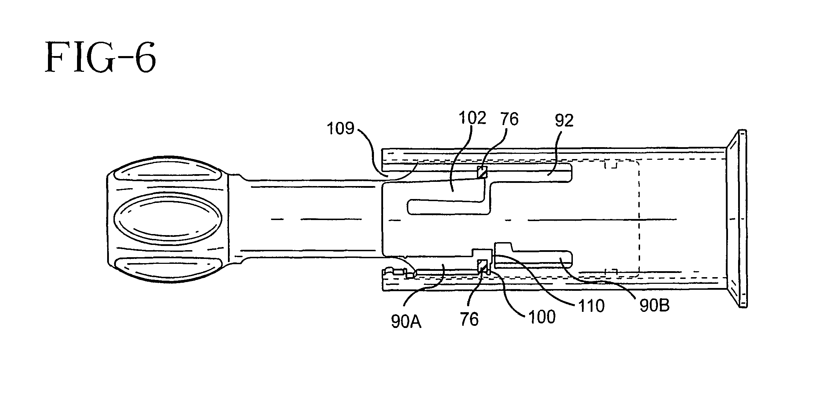

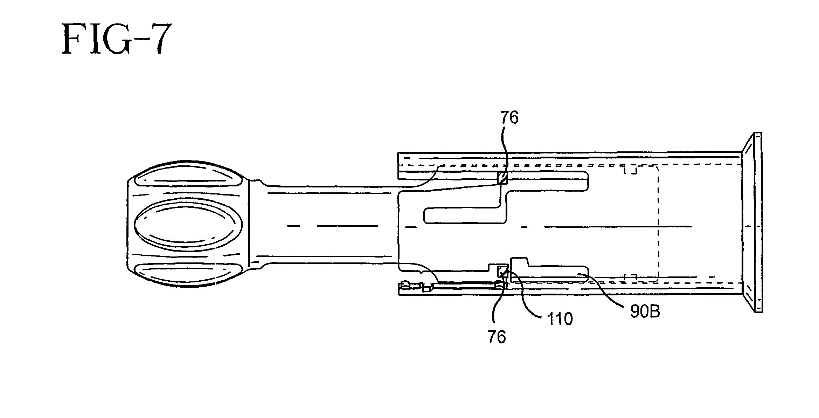

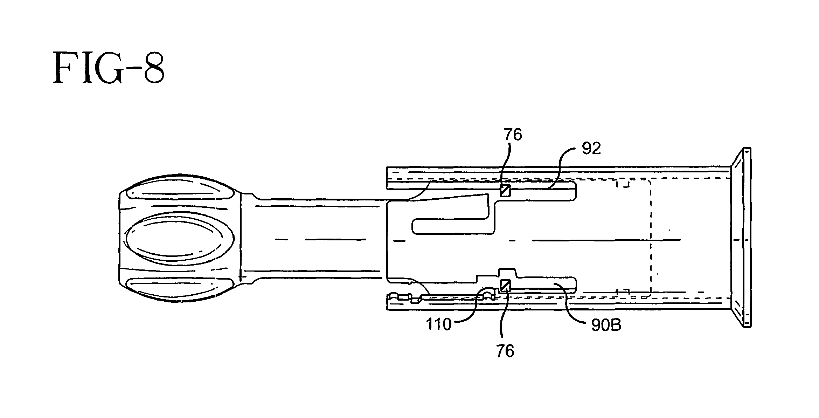

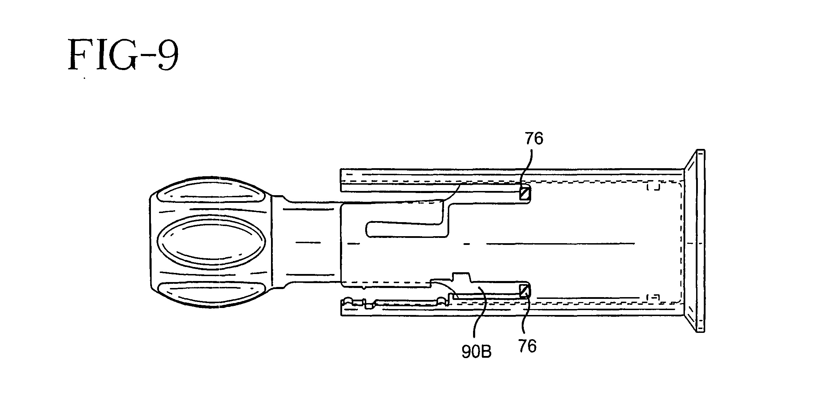

In addition, a corresponding rib 76 travels along the other or second slot 92 to provide structural stability and tracking, and the other slot 92 includes a biasing means to bias the rib in the first slot towards the second portion of the slot upon releasing/relaxing of the force applied by the user. Specifically, the other slot 92 includes a cut-away portion forming a deflectable arm 102 having a inner wall 104 associated with the slot slightly inclined and an upper portion 106 having a width less than a lower extending portion 108 and the slot 92 being wider at an open end 109 and narrowing along the slightly inclined inner wall 104 of the deflectable arm 102 (Figures 5 and 6). In this way, as the two ribs 72 travel along their respective slots 90, 92, the one rib traveling along slot 92 will deflect the flexible arm 102 to cause the proximal portion 28 of the holder to rotate relative to the distal portion 26 about its central axis, and the rib 76 traveling along slot 90 will move along the stop 100 and adjacent a bridge 110 similar to the first bridge but extending across the second portion 90B of the slot before traveling along the second portion 90B of the slot 90 (Figure 7). Accordingly, upon sufficient force being applied, the bridge 110 will fracture to allow passage of the rib 76 into the second portion 90B of the slot (Figure 8) and there along until it reaches the bottom of the slot (Figure 9). It should also be appreciated that, the inclined inner wall may have sufficient flexibility to provide the necessary biasing to cause relative rotation.

It should be appreciated that for purposes of illustration, the system has been described with respect to one set of slots and ribs. However, a corresponding arrangement is preferably provided on the other side of the holder. Also, it should be apparent that the slot can be divided into successive portions to further divide the substance to be delivered into additional doses.

Although the system of the present invention has been described in connection with preferably the use of a nasal syringe, it should be appreciated that the holder may also be used in connection with syringes having needles such as those described in U.S. Patent Nos. 4,964,866 (Szwarc), 4,986,818 (Imbert et al.) and 5,607,400 (Thibault et al.).

Also, it should be appreciated that although the preferred embodiment has been described in connection with a single compartment syringe, a multi-compartment syringe (not shown) can be utilized where, for example, a diluent is provided in one chamber and at least one dry or wet substance is provided in another chamber such as those described in U.S. Patent Nos. 4,599,082 (Grimard), 4,613,326 (Szwarc), 4,929,230 (Pfleger) and 4,235,235 (Bekkering). In this way, the first portion of the slot can be used to permit the stopper to travel a sufficient distance so as to permit mixing of the two substances. Thereafter, travel of the stopper permits the reconstituted substance to be delivered. Also, under such circumstances the initial bridge may be eliminated or reduced to a detent for merely attaching the two portions of the holder together.

The syringe 22 is preferably filled with the substance to be delivered and stoppered prior to assembly with the holder 24. In this way, the prefilled syringe can be inserted into the distal portion 26 of the holder and secured therein as a result of the rim of the syringe coming in contact with the detent 66 and in front of the grooves 64. Then the proximal portion 28 of the holder can be secured to the distal portion 26 of the holder by inserting the ribs 76 of the flanges 74 into the initial portion of each slot between the detents 99 and the bridges 98.

### Operation and Use

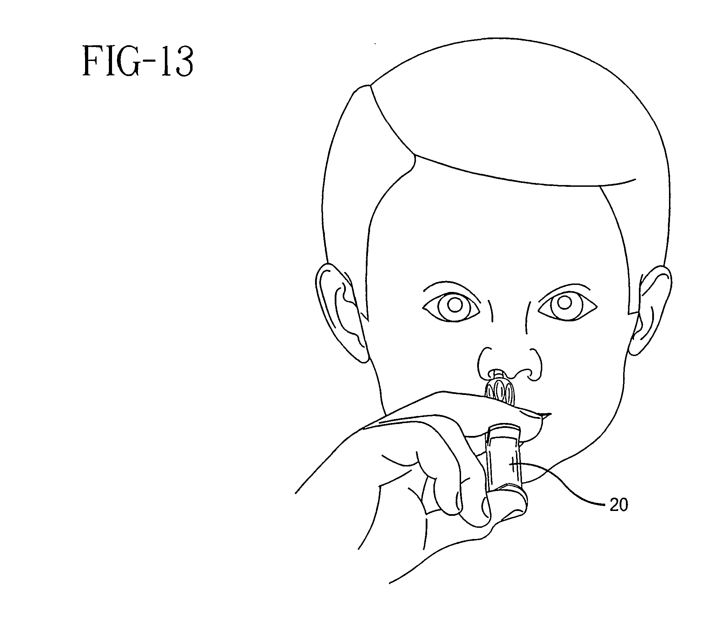

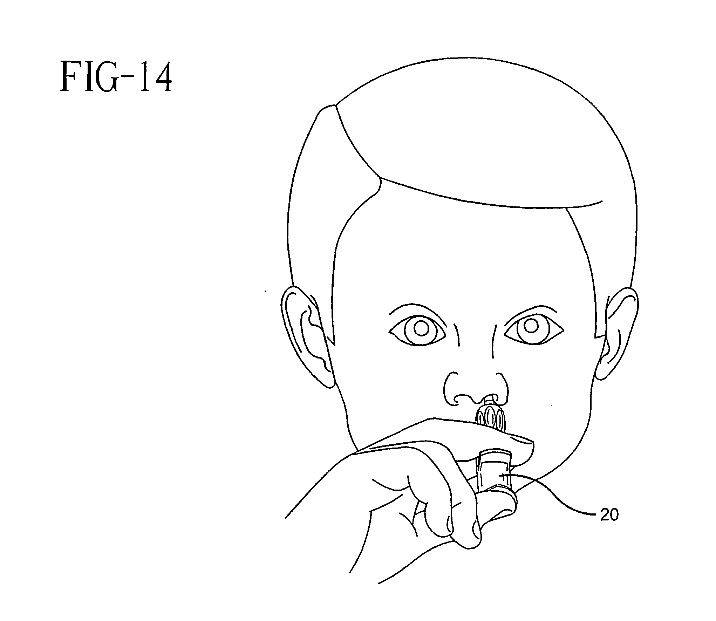

Having described the preferred embodiment of the drug delivery system 20 of the present invention, including the assembly thereof, its operation and use is described below in connection with Figures 5-12, and in particular Figures 13 and 14, with Figures 5 and 10 illustrating the system prior to activation.

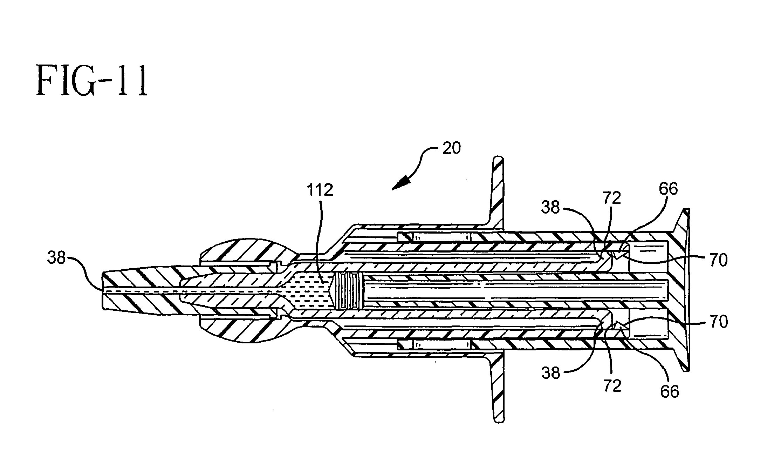

In operation, the user grasps the pre-assembled system 20 as shown in Figure 13 and with the tip cap 42 removed, insert the blunt tip into one nostril. The proximal portion of the holder is then moved towards the distal portion of the holder by the user squeezing together his thumb and two forefingers so that the two portions move towards each other with the proximal portion acting as a plunger rod (Figures 5 and 6). This movement, causes the displacement of the stopper 40 and expulsion of a predetermined amount of the substance 112 contained in the chamber of the syringe barrel through the orifice 37, depending upon the distance that the proximal portion, as well as the rod, travels until its progress is arrested by first stop, as shown in Figure 11. For simplicity, the total distance that the stopper 40 may be displaced into the barrel 30 of the syringe 22 is depicted by the legend "L" in Figure 10, with that distance "L" also correlating to the total quantity of substance retained in the syringe barrel. Similarly, the displacement of the stopper a distance L/n, would correlate to a first desired quantity of the substance to be administered from the syringe during a first motion of the proximal portion. For instance, for an application to a nasal syringe, it is typically desirable to ensure equal administration of the substance into each of the nostrils, meaning that it would be desirable to expel only half of the contents of the syringe at such time as the rib is arrested by the first stop (thus, L/n would correlate to L/2).

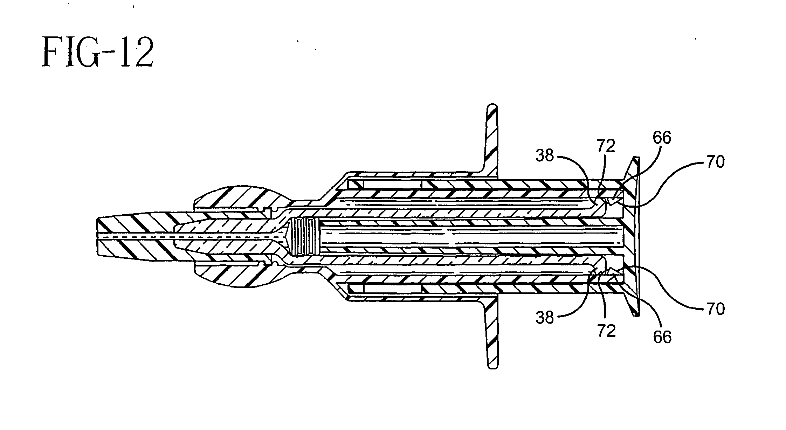

After a first administration of the substance, then the user would remove the system 20 from that nostril and place it in the other nostril (Figure 14), naturally relaxing the pressure or force being applied to the system resulting in the proximal portion of the holder rotating about its axis by the force exerted by the flexible arm so as to put the rib in position to travel along the second portion of the slot adjacent the second bridge (Figure 7). Once the rib is so situated and the system situated in the other nostril, the proximal portion of the holder is again squeezed, causing the rod to advance and fracture bridge 110 (Figure 8) and engage the stopper to expel the remaining contents of the syringe into the other nostril. Figures 9 and 12 show the system 20 once the contents have been expelled. Once so used, the entire system is typically discarded.

The system 20 provides a number of advantageous features. It allows the user to easily divide the dose to be delivered from the syringe using only one hand to position and activate the system. When used with a nasal syringe, the enlarged end of the holder limits the penetration of the syringe tip into the nostril. In addition, the overall configuration of the holder together with its enlarged flanges facilitates its handling and use with only one hand.

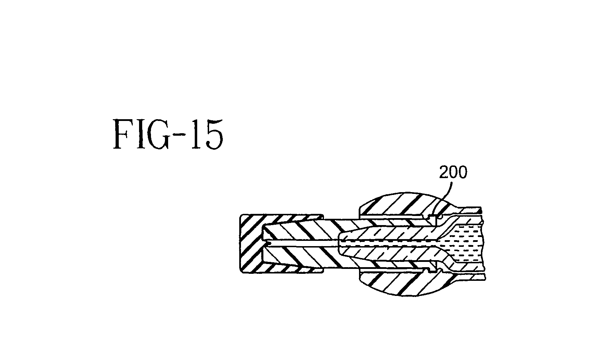

As illustrated in Figure 15, in the alternative, the syringe may be retained in the distal portion of the holder by a pair of detents or a groove 200 running along the tip in which a corresponding rim on the spray nozzle can be inserted.

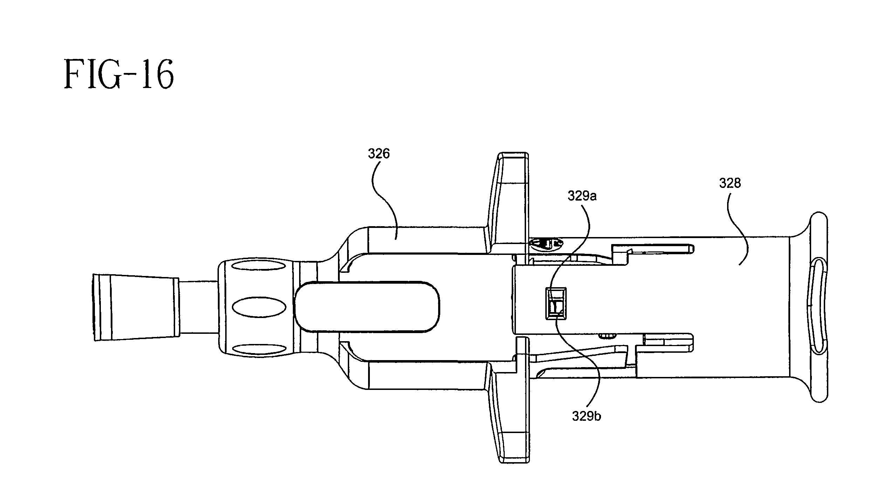

In the inventive embodiment illustrated in Figure 16, the distal portion 326 and the proximal portion 328 may be clipped together by means of flexible detents 329A and corresponding openings 329B, with the detents being situated on the distal portion and the corresponding openings being situated on the proximal portion so that when the two portions are assembled the detents project into the corresponding openings to attached the portions to one another. In this way, during assembly when the two portions are attached, there is less likelihood that the bridges will be broken and prematurely activate the system.

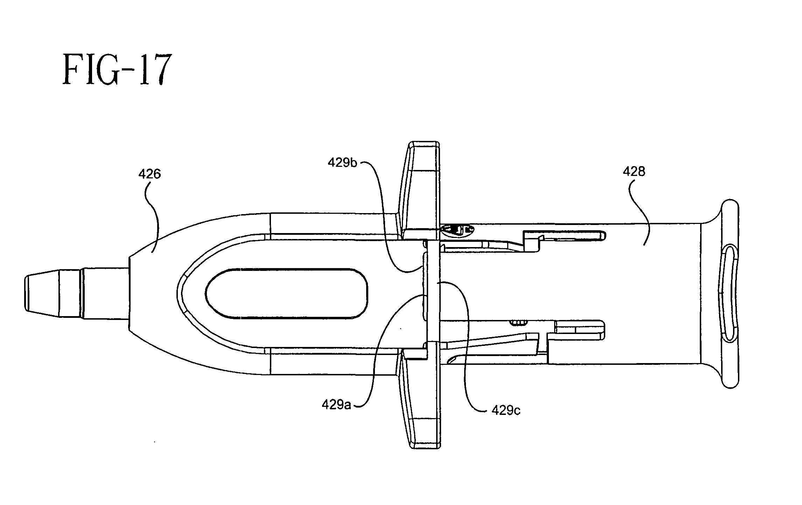

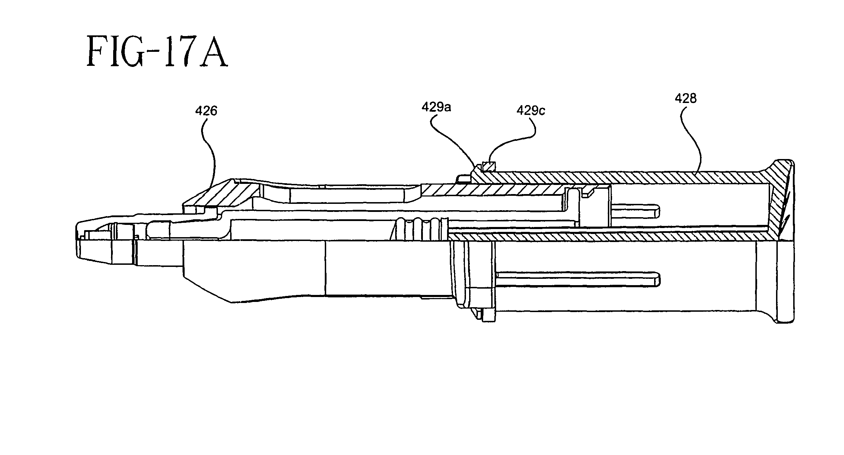

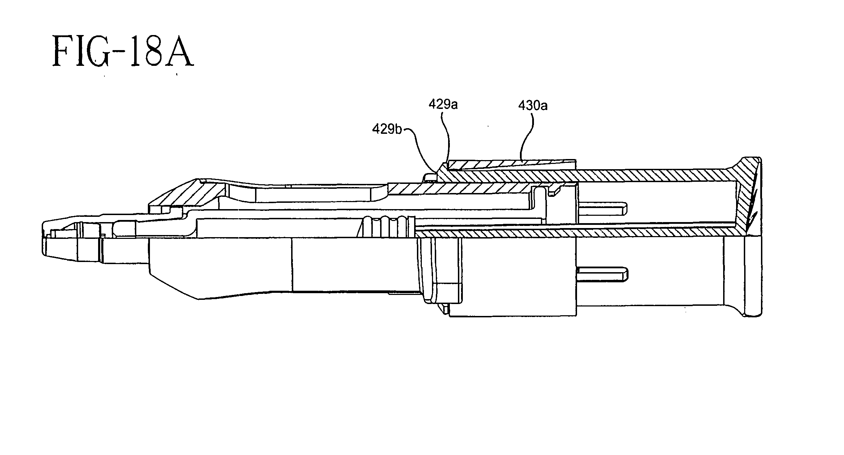

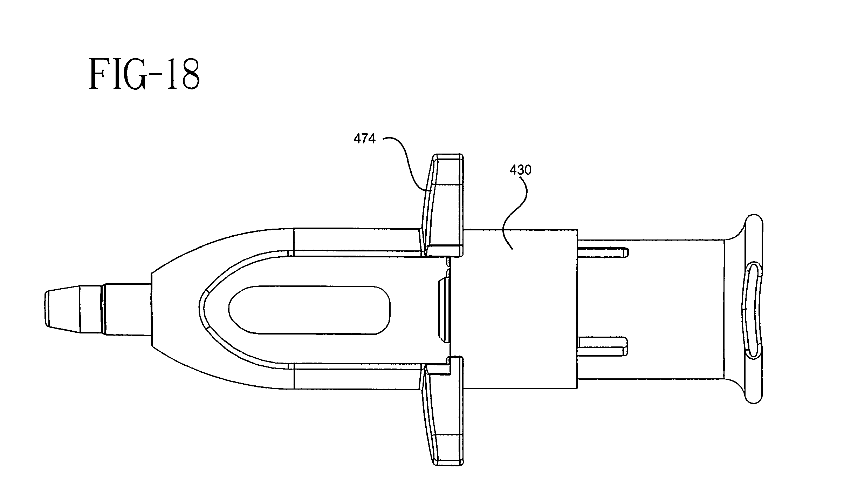

Alternatively as illustrated in Figure 17, and Figure 17A in greater detail, the distal portion 426 and the proximal portion 428 may be clipped together by means of flexible clips 429A and corresponding openings or gaps 429B, with the detents being situated on the proximal portion and the corresponding gaps being situated on the distal portion, with the gaps being formed by portions 429C of the distal portion bridging the space between the flanges. In this way, in addition to less likelihood of the bridges being broken, there is less friction caused during activation of the system. Also, as illustrated in Figure 18, and in greater detail in Figure 18A, a skirt 430A can be provided which extends along the bottom outwardly from the flanges 474, which can hide the various slots.

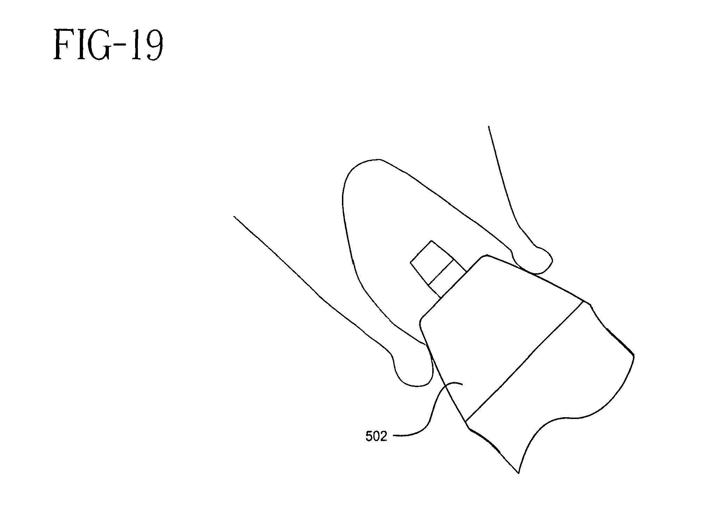

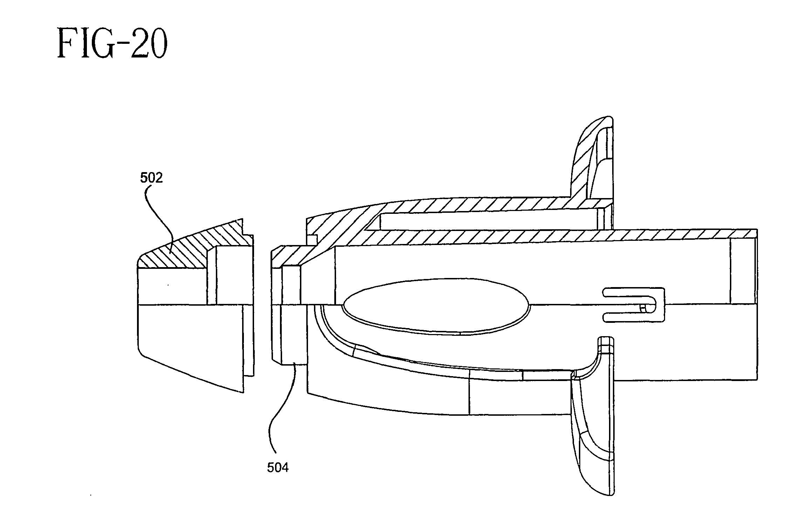

In another alternative embodiment as illustrated in Figures 19 and 20, a soft, flexible limiter 502 can be provided on the end of the system to limit the depth of insertion of the spray nozzle into the nostril, while providing added comfort and sealing of the nostril to avoid aerosol contamination. The limiter 502 may be snapped onto the tip 504 as illustrated in Figure 20, or co-injection molded as an integral component of the holder.

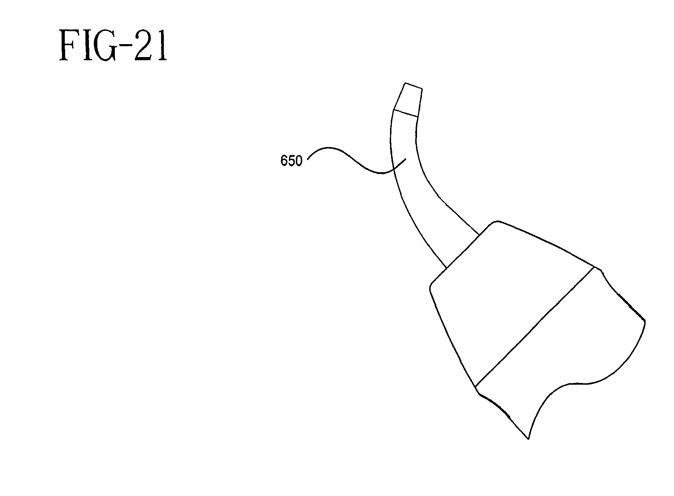

In an additional embodiment as illustrated in Figure 21, the spray nozzle 650 may be formed of a curved design to target specific areas in the nasal cavity. Alternatively, the spray nozzle may be attached to and form part of the holder (not shown).

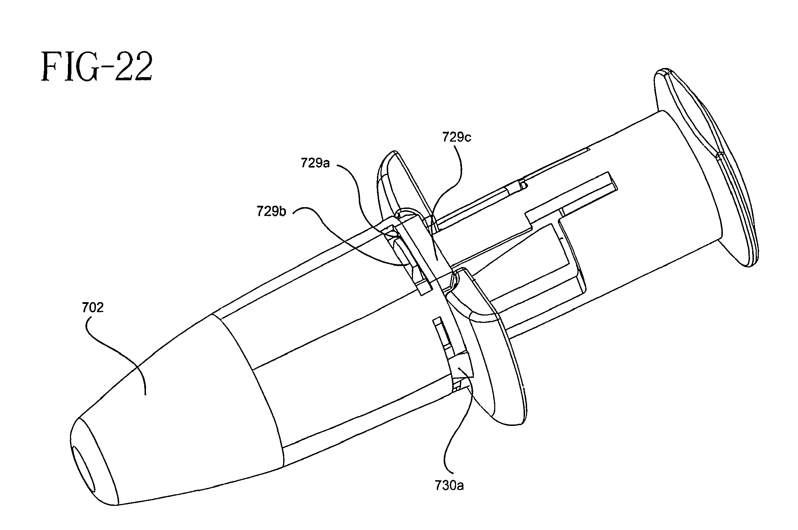

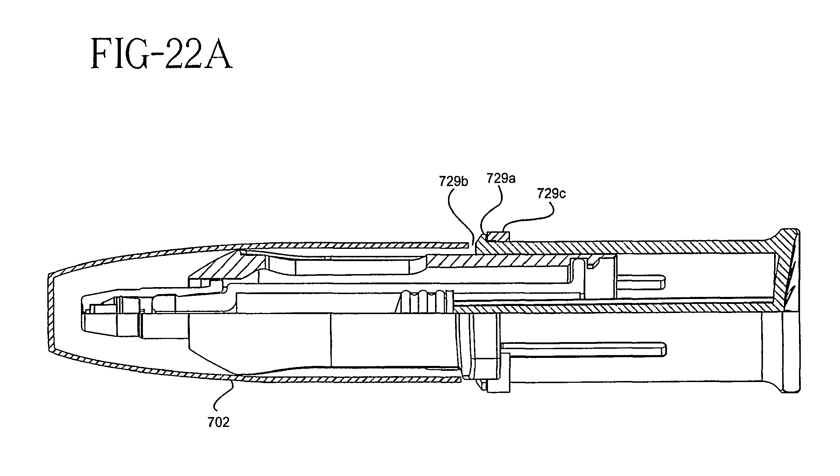

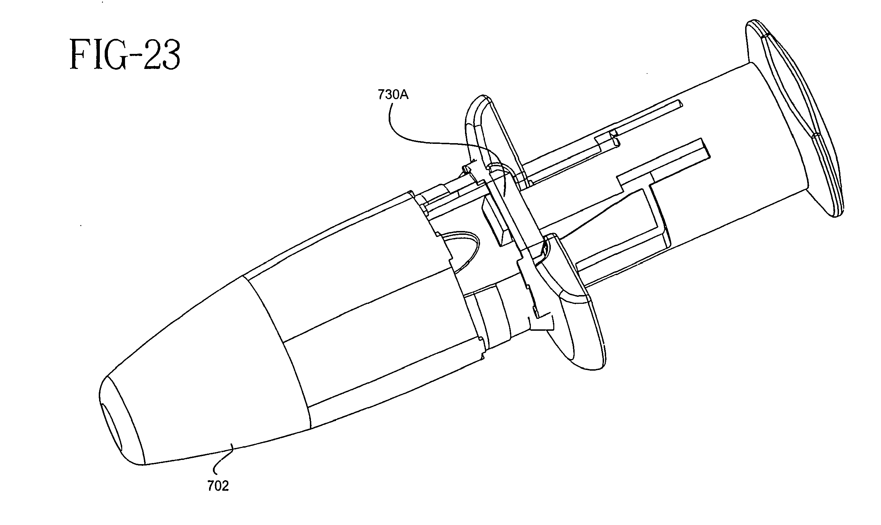

In yet another embodiment as illustrated in Figures 22 and 23, and in greater detail in Figure 22A, a cap 702 may be provided to the system, which can be clipped to the holder by means of flexible clips 729A and corresponding openings or gaps 729B, with the gaps being formed by a portion 729C of the cap extending across the space between the flanges of the distal portion, as well as provide tamper-evidence. In this way, in addition to less likelihood of the bridges being broken and less friction during activation of the system, the cap prevents activation of the system until the cap has been removed. To facilitate removal of the cap, frangible bridges 730A are provided which are fractured to remove the cap so that the system can be activated.

Accordingly, the system of the present invention may be used in a method to intranasally deliver various substance selected from the group consisting of drugs, vaccines and the like used in the prevention, alleviation, treatment, or cure of diseases. These substances may include: (i) drugs such as Anti-Angiogenesis agents, Antisense, anti-ulcer,butorphanol, Calcitonin and analogs, COX-II inhibitors, desmopressin and analogs, dihydroergotamine, Dopamine agonists and antagonists, Enkephalins and other opioid peptides, Growth hormone and analogs (including growth hormone releasing hormone), Growth hormone antagonists, IgE suppressors, Insulin, insulinotropin and analogs, Ketamine, Kytril, Lutenizing hormone releasing hormone and analogs, lidocaine, metoclopramide, Midazolam, Narcotic analgesics, neuraminidase inhibitors, nicotine, Non-steroid anti-inflammatory agents, Oligosaccharides, ondansetron, Parathyroid hormone and analogs, Parathyroid hormone antagonists, Prostaglandin antagonists, Prostaglandins, Recombinant soluble receptors, scopolamine, Serotonin agonists and antagonists, Sildenafil, Terbutaline, vasopressin; (ii) vaccines with or without carriers/adjuvants such as prophylactics and therapeutic antigens (including but not limited to subunit protein, peptide and polysaccharide, polysaccharide conjugates, toxoids, genetic based vaccines, live attenuated, reassortant, inactivated, whole cells, viral and bacterial vectors) in connection with, arthritis, cholera, cocaine addiction, HIB, meningococcus, measles, mumps, rubella, varicella, yellow fever, Respiratory syncytial virus, pneumococcus, streptococcus, typhoid, influenza, hepatitis, including hepatitis A, B, C and E, polio, HIV, parainfluenza, rotavirus, CMV, chlamydia, non-typeable haemophilus, moraxella catarrhalis, human papilloma virus, tuberculosis including BCG, gonorrhoea, asthma, atheroschlerosis, malaria, otitis media, E-coli, Alzheimers, H. Pylori, salmonella, diabetes, cancer and herpes simplex; and (iii) other substances in all of the major therapeutics such as Agents for the common cold, Anti-addiction, anti-infectives, analgesics, anesthetics, anorexics, antiarthritics, anti-allergy agents, antiasthmatic agents, anticonvulsants, anti-depressants, antidiabetic agents, anti-depressants, anti-diuretics, anti-emetics, antihistamines, anti-inflammatory agents, antimigraine preparations, antimotion sickness preparations, antinauseants, antineoplastics, anti-obesity, antiosteoporeteic, antiparkinsonism drugs, antipruritics, antipsychotics, antipyretics, anticholinergics, benzodiazepine antagonists, bone stimulating agents, central nervous system stimulants, hormones, hypnotics, immunosuppressives, prostaglandins, proteins, peptides, polypeptides and other macromolecules, psychostimulants, rhinitis treatment, sedatives, sexual hypofunction, tranquilizers and vitamins including B 12.

The present invention is suited for being used with the above substances in the preparation of a filled device for intranasally delivering the various substance. The actual filling or insertion of the desired substance into the syringe can be accomplished in any of several known manners. Example preparation and filling techniques are disclosed in U.S. Patent Nos. 6,164,044 to Profano et al., 6,189,292 to Odell et al., 5,620,425 to Hefferman et al.; 5,597,530 to Smith et al.; 5,537,042 to DeHaen; 5,531,255 to Vacca; 5,519,984 to Veussink et al.; 5,373,684 to Veussink et al.; 5,265,154 to Liebert et al.; 5,287,983 to Liebert et al.; and 4,718,463 to Jurgens, Jr. et al..

While the preferred embodiment of the present invention has been described so as to enable one skilled in the art to practice the system of the present invention, it is to be understood that variations and modifications may be employed without departing from the concept and intent of the present invention as defined in the following claims. The preceding description is intended to be exemplary and should not be used to limit the scope of the invention. The scope of the invention should be determined only by reference to the following claims.

## Claims

1. A system (20) for delivering at least one substance in at least two doses, comprising:
a drug container (22) including a barrel (30), a first end extending from said barrel, and a stopper (40) slidably positioned within said barrel;
a holder (24, 324, 424) having a distal portion (26, 326, 426) and a proximal portion (28, 328, 428), with the distal portion being assembled to the proximal portion, with the drug container secured therein; and
means for controlling the delivery of a substance contained in the barrel of the drug container including a plurality of slots (90, 92) extending axially along at least one of said portions of the holder whereby upon activation of said system, said portions of said holder move towards one another upon the application of a minimum force and said stopper (40) moves a preselected axial distance to expel at least a portion of said substance from said drug container, comprising:
a pair of exterior ribs (76) on said distal portion (26, 326, 426) of said holder (24, 324, 424), a pair of flanges (74) attached to said ribs (76), wherein said slots (90, 92) in said proximal portion of said holder include at least one pair of slots situated thereon, with said pair of slots including a first slot (90) and a second slot (92) extending axially along the body of the proximal portion of the holder generally parallel to each other and dimensioned and situated to accommodate the ribs (76) so that one of said ribs is insertable into each slot and able to travel along the slot upon activation of the system,
wherein said distal portion and said proximal portion of said holder are attached to one another by means of flexible members (329A, 429A) and corresponding openings (329B, 429B) to avoid premature activation of the system.

2. The system as described in claim 1, wherein said openings (329B, 429B) are formed by a portion (429C) of said distal portion bridging a space between a pair of flanges (74, 474) extending radially outwardly form said distal portion.

3. The system as described in claim 2, wherein said distal portion (26, 326, 426) includes a skirt (430A) extending from the distal portion, including said bridging portion (429C), and covering the means for controlling the delivery of the substance.

4. The system as described in claim 1, wherein said first end of said drug container includes a spray nozzle (50, 650) for use in intranasally delivering the substance and said drug container is a syringe.

5. The system as described in claim 4, further comprising a limiter (502) associated with a first end of said distal portion, and said limiter limiting the depth of insertion of the spray nozzle into a nostril.

6. The system as described in claim 5, wherein said limiter (502) is formed of a curved design to target specific areas in a nasal cavity.

7. The system as described in claim 4, wherein said spray nozzle (50) is formed of a curved design to target specific areas in a nasal cavity.

8. The system as described in claim 1, further comprising a cap (702) covering at least a first end of said distal portion.

9. The system as described in claim 8, wherein said cap (702) includes an opening (729B) being formed by a portion of the cap extending across a space between the flanges.

10. The system as described in claim 8, wherein said cap includes a tamper evident means (729A, 729B).

11. The system as described in claim 1, wherein said distal portion (26, 326, 426) and said proximal portion (28, 328, 428) of said holder (24, 324, 424) each has a generally tubular interior configured to accommodate said drug container filled with a substance to be delivered and said proximal portion of said holder includes a closed end (82) having a rod (84) extending therefrom for engagement with said stopper of said drug container upon activation.

12. The system as described in claim 1, wherein said preselected axial distance corresponds to about a dosage of the substance held in said drug container barrel desired to be administered in a first motion of said stopper.

13. The system as described in claim 12, wherein said preselected axial distance corresponds to about half the distance that said stopper is capable of moving within said barrel to administer about half of the substance held by said drug container.

14. The system as described in claim 1, said system further comprising a means (64, 66) for securing said drug container in said distal portion (26, 326, 426) of said holder with said distal portion having a first end (60) through which the first end of said drug container can extend and a second, open end (62) defining an opening of sufficient size for receiving the drug container.

15. The system as described in claim 14, wherein said drug container is a syringe having a rim extending from an open end thereof and said drug container securing means (64, 66) is situated adjacent said open end (62) of said distal portion and includes at least one detent (66) situated adjacent the open end and dimensioned so that the rim of the syringe may be securely retained in said distal portion by said detent.

16. The system as described in claim 1, further comprising a pair of flanges (74, 474) extending radially outwardly from said distal portion of said holder and attached thereto by a plurality of ribs (76).

17. The system as described in claim 1, wherein the first slot (90) is preferably open and is divided into at least two portions (90A, 90B), and situated adjacent an open end of the first slot is a bridge (98) extending across at least a portion of the slot, with the bridge being dimensioned so that when the rib (76) comes in contact with the bridge and sufficient force is applied there against, the bridge will fracture to allow passage of the rib along the slot.

18. The system as described in claim 17, wherein a detent (99) is situated adjacent the open end (80) of the first portion (90A) of the first slot (90) so that the rib (76) can be clipped between the detent and the bridge (98) prior to activation of the system, and the second portion (90B) of the first slot (90) is at least slightly offset from the first portion (90A) of the first slot (90) and towards the second slot (92).

19. The system as described in claim 18, wherein the other rib (76) travels along the second slot (92) to provide structural stability and tracking, with the second slot (92) including biasing means (102) for biasing the rib in the first slot towards the second portion of the slot upon release of the force applied by a user.

20. The system as described in claim 19, wherein said biasing means is adapted to include a cut-away portion forming a deflectable arm (102) having an inner wall (104) associated with the second slot (92) so that as the ribs (76) travel along their respective slots, the one rib will deflect the flexible arm (102) to cause the proximal portion (28, 328, 428) of the holder to rotate relative to the distal portion (26, 326, 426) about a central axis so that the rib situated in the first slot (90) can come in contact with a second bridge (110) so that upon sufficient force being applied, the bridge (110) will fracture to allow passage of the rib along the second portion of the first slot.

21. The system as described in claim 1, wherein said distal portion (26, 326, 426) of said holder includes at least one window (78) to permit visual inspect of the contents of the drug container located within the holder.

22. A holder (24) for the drug container of one of claims 1-21, comprising:
a distal portion (26, 326, 426) and a proximal portion (28, 328, 428), each having configured to accommodate a drug container (22) filled with a substance to be delivered, with the distal portion being able to be assembled to the proximal portion; said distal portion comprising a pair of exterior ribs (76) and a pair of flanges (74) attached to said ribs (76);
means for controlling the delivery of the substance including a plurality of slots (90, 92) extending axially along at least one of said portions of the holder and situated to accomodate the ribs so that one of said ribs is insertable into each slot and able to travel along the slot upon activation of the system, whereby when said portions of said holder are moved towards one another a preselected axial distance upon the application of a minimum force, at least a portion of the substance can be expelled from said drug container, wherein said distal portion and said proximal portion of said holder are attached to one another by means of flexible members (329A, 429A) and corresponding openings (329B, 429B) to avoid premature activation of the system.

23. The holder as described in claim 22, wherein a first end of said drug container includes a spray nozzle (50) and said drug container is a syringe, and said distal portion (26, 326, 426) and said proximal portion (28, 328, 428) each has a generally tubular interior configured to accommodate said syringe and said proximal portion of said holder (24) includes a closed end (82) having a rod (84) extending therefrom for engagement with said stopper of said syringe during activation.

24. The holder as described in claim 22, wherein said preselected axial distance corresponds to about a dosage of the substance held in said drug container desired to be administered.

25. The holder as described in claim 24, wherein said distal portion (26, 326, 426) further comprises a means (64, 66) for securing said drug container therein with said distal portion having a first end (60) through which a first end of said drug container can extend and a second, open end (62) defining an opening of sufficient size for receiving a barrel of the drug container.

26. The holder as described in claim 25, further comprising a pair of flanges (74, 474) extending radially outwardly from said distal portion and attached there along by the ribs (76).

27. The holder as described in claim 26, wherein said slots (90, 92) in said proximal portion of said holder include at least one pair of slots situated thereon, with said pair of slots including a first slot (90) and a second slot (92) extending axially along the body of the proximal portion of the holder generally parallel to each other and dimensioned and situated to accommodate the ribs so that the ribs are insertable into the slots and able to travel along the slots upon activation of the system.

28. The holder as described in claim 27, wherein the first slot (90) is preferably open and is divided into at least two portions (90A, 90B), and situated adjacent an open end of the first slot is a bridge (98) extending across at least a portion of the slot, with the bridge being dimensioned so that when a rib (76) comes in contact with the bridge and sufficient force is applied there against, the bridge will fracture to allow passage of the rib along the slot.

29. The holder as described in claim 28, wherein a detent (99) is situated adjacent said open end (80) of the first portion (90A) of the first slot (90) so that the rib (76) can be clipped between the detent and the bridge (98) prior to activation of the system, and the second portion (90B) of the first slot (90) is at least slightly offset from the first portion (90A) of the first slot (90) and towards the second slot (92).

30. The holder as described in claim 29, wherein one of said ribs (76) travels along the second slot (92) to provide structural stability and tracking, with the second slot including biasing means (102) for biasing the rib (76) in the first slot (90) towards the second portion (90B) of the slot upon release of the force applied by a user.

31. The holder as described in claim 22, wherein said distal portion (26, 326, 426) includes at least one window (78) to permit visual inspect of the contents of the drug container when located within the holder.

32. The holder as described in claim 22, wherein said openings (329B, 429B) are formed by a portion (429C) of said distal portion bridging a space between a pair of flanges (74, 474) extending radially outwardly form said distal portion.

33. The holder as described in claim 32, wherein said distal portion (26, 326, 426) includes a skirt (430A) extending from the distal portion, including said bridging portion (429C), and covering the means for controlling the delivery of the substance.

34. The holder as described in claim 22, wherein said first end of said drug container includes a spray nozzle (50, 650) for use in intranasally delivering the substance and said drug container is a syringe.

35. The holder as described in claim 34, further comprising a limiter (502) associated with a first end of said distal portion, and said limiter limiting the depth of insertion of the spray nozzle into a nostril.

36. The holder as described in claim 35, wherein said limiter (502) is formed of a curved design to target specific areas in a nasal cavity.

37. The holder as described in claim 34, wherein said spray nozzle (50, 650) is formed of a curved design to target specific areas in a nasal cavity.

38. The holder as described in claim 22, further comprising a cap (702) covering at least a first end of said distal portion.

39. The holder as described in claim 38, wherein said cap (702) includes an opening (729B) being formed by a portion of the cap extending across a space between the flanges.

40. The holder as described in claim 38, wherein said cap includes a tamper evident means (729A, 729B).

## Patentansprüche

1. System (20) zum Zuführen mindestens einer Substanz in mindestens zwei Dosen, mit:
einem Arzneimittelbehälter (22) mit einem Zylinder (30), einem von dem Zylinder abstehenden ersten Ende und einem gleitbar in dem Zylinder positionierten Stopfen (40);
einem Halter (24,324,424) mit einem distalen Teil (26,326,426) und einem proximalen Teil (28,328,428), wobei der distale Teil mit dem proximalen Teil zusammengesetzt ist und der Arzneimittelbehälter darin angeordnet ist; und
einer Einrichtung zum Steuern der Zuführung einer in dem Zylinder des Arzneimittelbehälters enthaltenen Substanz mit mehreren axial entlang mindestens einem der Teile des Halters verlaufenden Schlitzen (90,92), wobei sich bei Aktivierung des Systems die Teile des Behälters bei Aufbringen einer minimalen Kraft aufeinander zu bewegen und sich der Stopfen (40) um eine vorgewählte axiale Strecke bewegt, um mindestens einen Teil der Substanz aus dem Arzneimittelbehälter auszugeben,
wobei das System aufweist:
ein Paar Außenrippen (76) am distalen Teil (26,326,426) des Halters (24,324,424) und ein Paar mit den Rippen (76) verbundene Flansche (74), wobei die Schlitze (90,92) in dem proximalen Teil des Halters mindestens ein Paar auf dem proximalen Teil befindliche Schlitze aufweist, das Paar Schlitze einen ersten Schlitz (90) und einen zweiten Schlitz (92) aufweist, die axial entlang dem Körper des proximalen Teils des Halters und im Wesentlichen parallel zueinander verlaufen und derart dimensioniert und angeordnet sind, dass sie die Rippen (76) derart aufnehmen, dass eine der Rippen in jeden Schlitz einsetzbar und in der Lage ist, sich bei Aktivierung des Systems den Schlitz entlang zu bewegen,
wobei der distale Teil und der proximale Teil des Halters über flexible Elemente (329A,429A) und entsprechende Öffnungen (329B,429B) miteinander verbunden sind, um eine vorzeitige Aktivierung des Systems zu verhindern.

2. System nach Anspruch 1, bei dem die Öffnungen (329B,429B) von einem Teil (429C) des distalen Teils gebildet sind, der einen Zwischenraum zwischen einem Paar Flansche (74,474) überbrückt, welche von dem distalen Teil radial nach außen abstehen.

3. System nach Anspruch 2, bei dem der distale Teil (26,326,426) eine von dem distalen Teil abstehende Einfassung (430A) aufweist, die den Überbrückungsteil (429C) aufweist und die Einrichtung zum Steuern der Zuführung der Substanz abdeckt.

4. System nach Anspruch 1, bei dem das erste Ende des Arzneimittelbehälters eine Sprühdüse (50,650) zur Verwendung bei der intranasalen Zuführung der Substanz aufweist und der Arzneimittelbehälter eine Spritze ist.

5. System nach Anspruch 4, ferner mit einem mit einem ersten Ende des distalen Teils verbundenen Begrenzungsteil (502), der die Einsetztiefe der Sprühdüse in ein Nasenloch begrenzt.

6. System nach Anspruch 5, bei dem der Begrenzungsteil (502) gekrümmt ist, um auf spezifische Bereiche in einer Nasenhöhle zu zielen.

7. System nach Anspruch 4, bei dem die Sprühdüse (50) gekrümmt ist, um auf spezifische Bereiche in einer Nasenhöhle zu zielen.

8. System nach Anspruch 1, ferner mit einer Kappe (702), die mindestens ein erstes Ende des distalen Teils abdeckt.

9. System nach Anspruch 8, bei dem die Kappe (702) eine Öffnung (729B) aufweist, die von einem Teil der Kappe gebildet ist, der über einen Zwischenraum zwischen den Flanschen verläuft.

10. System nach Anspruch 8, bei dem die Kappe eine Missbrauchserkennungseinrichtung (729A,729B) aufweist.

11. System nach Anspruch 1, bei dem der distale Teil (26,326,426) und der proximale Teil (28,328,428) des Halters (24,324,424) jeweils einen im Wesentlichen rohrförmigen Innenraum aufweisen, der zum Aufnehmen des mit einer zuzuführenden Substanz gefüllten Arzneimittelbehälters vorgesehen ist, und der proximale Endteil des Halters ein geschlossenes Ende (82) mit einem Stab (84) aufweist, der zwecks Zusammengreifens mit dem Stopfen des Arzneimittelsbehälters bei Aktivierung von dem Ende (82) absteht.

12. System nach Anspruch 1, bei dem die vorgewählte axiale Strecke ungefähr einer Dosis der in dem Arzneimittelbehälterzylinder enthaltenen Substanz entspricht, die bei einer ersten Bewegung des Stopfens verabreicht werden soll.

13. System nach Anspruch 12, bei dem die vorgewählte axiale Strecke ungefähr der halben Strecke entspricht, über die sich der Stopfen in dem Zylinder bewegen kann, um ungefähr die Hälfte der in dem Arzneimittelbehälter enthaltenen Substanz zu verabreichen.

14. System nach Anspruch 1, wobei das System ferner eine Einrichtung (64,66) zum Sichern des Arzneimittelbehälters in dem distalen Teil (26, 326,426) des Halters aufweist, wobei der distale Teil ein erstes Ende (60), durch das das erste Ende des Arzneimittelbehälters verlaufen kann, und ein zweites offenes Ende (62) aufweist, das eine Öffnung mit ausreichender Größe zum Aufnehmen des Arzneimittelbehälters begrenzt.

15. System nach Anspruch 14, bei dem der Arzneimittelbehälter eine Spritze mit einem von einem offenen Ende der Spritze abstehenden Rand ist und die Arzneimittelbehälter-Sicherungseinrichtung (64,66) benachbart zu dem offenen Ende (62) des distalen Teils angeordnet ist und mindestens eine Raste (66) aufweist, die benachbart zu dem offenen Ende angeordnet und derart dimensioniert ist, dass der Rand der Spritze von der Raste sicher in dem distalen Teil gehalten werden kann.

16. System nach Anspruch 1, ferner mit einem Paar Flansche (74,474), die von dem distalen Teil des Halters radial nach außen abstehen und über mehrere Rippen (76) mit dem Halter verbunden sind.

17. System nach Anspruch 1, bei dem der erste Schlitz (90) vorzugsweise offen und in mindestens zwei Teile (90A,90B) unterteilt ist und benachbart zu einem offenen Ende des ersten Schlitzes eine Brücke (98) vorgesehen ist, die über mindestens einen Teil des Schlitzes verläuft, wobei die Brücke derart dimensioniert ist, dass dann, wenn die Rippe (76) mit der Brücke in Kontakt kommt und eine ausreichende Kraft aufgebracht wird, die Brücke bricht, um ein Durchtreten der Rippe entlang dem Schlitz zu ermöglichen.

18. System nach Anspruch 17, bei dem eine Raste (99) derart benachbart zu dem offenen Ende (80) des ersten Teils (90A) des ersten Schlitzes (90) angeordnet ist, dass die Rippe (76) vor Aktivierung des Systems zwischen der Raste und der Brücke (98) festgeklemmt werden kann, und der zweite Teil (90B) des ersten Schlitzes (90) zumindest leicht von dem ersten Teil (90A) des ersten Schlitzes (90) in Richtung auf den zweiten Schlitz (92) versetzt ist.

19. System nach Anspruch 18, bei dem die andere Rippe (76) zwecks Verleihung einer strukturellen Stabilität und zwecks Nachführung den zweiten Schlitz (92) entlang verläuft, wobei der zweite Schlitz (92) eine Vorspanneinrichtung (102) zum Vorspannen der Rippe in dem ersten Schlitz in Richtung auf den zweiten Teil des Schlitzes bei Wegnahme des von einem Benutzer aufgebrachten Kraft aufweist.

20. System nach Anspruch 19, bei dem die Vorspanneinrichtung einen Ausnehmungsteil aufweist, der einen biegbaren Arm (102) mit einer dem zweiten Schlitz (92) zugeordneten Innenwand (104) bildet, so dass beim Laufen der Rippen (76) entlang ihren jeweiligen Schlitzen die eine Rippe den flexiblen Arm (102) biegt, um zu bewirken, dass sich der proximale Teil (28,328,428) des Halters relativ zu dem distalen Teil (26,326,426) derart um eine Mittelachse dreht, dass die in dem ersten Schlitz (90) befindliche Rippe mit einer zweiten Brücke (110) derart in Kontakt kommen kann, dass bei Aufbringen einer ausreichenden Kraft die Brücke (110) bricht, um ein Durchtreten der Rippe entlang dem ersten Teil des ersten Schlitzes zu ermöglichen.

21. System nach Anspruch 1, bei dem der distale Teil (26,326,426) des Halters mindestens ein Fenster (78) aufweist, um eine Sichtkontrolle des Inhalts des in dem Halter befindlichen Arzneimittelbehälters zu ermöglichen.

22. Halter (24) für den Arzneimittelbehälter nach einem der Ansprüche 1-21, mit:
einem distalen Teil (26,326,426) und einem proximalen Teil (28,328, 428), die jeweils zum Aufnehmen eines mit einer zuzuführenden Substanz gefüllten Arzneimittelbehälters (22) konfiguriert sind, wobei der distale Teil mit dem proximalen Teil zusammengesetzt werden kann; und der distale Teil ein Paar Außenrippen (76) und ein Paar mit den Rippen (76) verbundene Flansche (74) aufweist;
eine Einrichtung zum Steuern der Zuführung der Substanz mit mehreren Schlitzen (90,92), die axial entlang mindestens einem der Teile des Halters verlaufen und zum Aufnehmen der Rippen derart vorgesehen sind, dass eine der Rippen in jeden Schlitz einsetzbar und in der Lage ist, sich bei Aktivierung des Systems den Schlitz entlang zu bewegen,
wobei dann, wenn die Teile des Halters bei Aufbringen einer minimalen Kraft über eine vorgewählte axiale Strecke aufeinander zu bewegt werden, mindestens ein Teil der Substanz aus dem Arzneimittelbehälter ausgegeben werden kann, wobei der distale Teil und der proximale Teil des Halters über flexible Elemente (329A,429A) und entsprechende Öffnungen (329B,429B) miteinander verbunden sind, um eine vorzeitige Aktivierung des Systems zu verhindern.

23. Halter nach Anspruch 22, bei dem ein erstes Ende des Arzneimittelbehälters eine Sprühdüse (50) aufweist und der Arzneimittelbehälter eine Spritze ist und der distale Teil (26,326,426) und der proximale Teil (28, 328,428) jeweils einen im Wesentlichen rohrförmigen Innenraum aufweisen, der zum Aufnehmen der Spritze vorgesehen ist, und der proximale Teil des Halters (24) ein geschlossenes Ende (82) mit einem Stab (84) aufweist, der zwecks Zusammengreifens mit dem Stopfen der Spritze bei Aktivierung von dem Ende (82) absteht.

24. Halter nach Anspruch 22, bei dem die vorgewählte axiale Strecke ungefähr einer Dosis der in dem Arzneimittelbehälter enthaltenen zu verabreichenden Substanz entspricht.

25. Halter nach Anspruch 24, bei dem der distale Teil (26,326,426) ferner eine Einrichtung (64,66) zum Sichern des Arzneimittelbehälters in dem distalen Teil (26,326,426) aufweist, wobei der distale Teil ein erstes Ende (60), durch das ein erstes Ende des Arzneimittelbehälters verlaufen kann, und ein zweites offenes Ende (62) aufweist, das eine Öffnung mit ausreichender Größe zum Aufnehmen eines Zylinders des Arzneimittelbehälters begrenzt.

26. Halter nach Anspruch 25, ferner mit einem Paar Flanschen (74,474), die von dem distalen Teil radial nach außen abstehen und über mehrere Rippen (76) mit dem distalen Teil verbunden sind.

27. Halter nach Anspruch 26, bei dem die Schlitze (90,92) in dem proximalen Teil des Halters mindestens ein Paar auf dem proximalen Teil befindliche Schlitze aufweist, wobei das Paar Schlitze einen ersten Schlitz (90) und einen zweiten Schlitz (92) aufweist, die axial entlang dem Körper des proximalen Teils des Halters und im Wesentlichen parallel zueinander verlaufen und derart dimensioniert und angeordnet sind, dass sie die Rippen derart aufnehmen, dass die Rippen in die Schlitze einsetzbar und in der Lage sind, sich bei Aktivierung des Systems die Schlitze entlang zu bewegen.

28. Halter nach Anspruch 27, bei dem der erste Schlitz (90) vorzugsweise offen und in mindestens zwei Teile (90A,90B) unterteilt ist und benachbart zu einem offenen Ende des ersten Schlitzes eine Brücke (98) vorgesehen ist, die über mindestens einen Teil des Schlitzes verläuft, wobei die Brücke derart dimensioniert ist, dass dann, wenn die Rippe (76) mit der Brücke in Kontakt kommt und eine ausreichende Kraft aufgebracht wird, die Brücke bricht, um ein Durchtreten der Rippe entlang dem Schlitz zu ermöglichen.

29. Halter nach Anspruch 28, bei dem eine Raste (99) derart benachbart zu dem offenen Ende (80) des ersten Teils (90A) des ersten Schlitzes (90) angeordnet ist, dass die Rippe (76) vor Aktivierung des Systems zwischen der Raste und der Brücke (98) festgeklemmt werden kann, und der zweite Teil (90B) des ersten Schlitzes (90) zumindest leicht von dem ersten Teil (90A) des ersten Schlitzes (90) in Richtung auf den zweiten Schlitz (92) versetzt ist.

30. Halter nach Anspruch 29, bei dem eine der Rippen (76) zwecks Verleihung einer strukturellen Stabilität und zwecks Nachführung den zweiten Schlitz (92) entlang verläuft, wobei der zweite Schlitz eine Vorspanneinrichtung (102) zum Vorspannen der Rippe (76) in dem ersten Schlitz (90) in Richtung auf den zweiten Teil (90B) des Schlitzes bei Wegnahme des von einem Benutzer aufgebrachten Kraft aufweist.

31. Halter nach Anspruch 22, bei dem der distale Teil (26,326,426) mindestens ein Fenster (78) aufweist, um eine Sichtkontrolle des Inhalts des in dem Halter befindlichen Arzneimittelbehälters zu ermöglichen.

32. Halter nach Anspruch 22, bei dem die Öffnungen (329B,429B) von einem Teil (429C) des distalen Teils gebildet sind, der einen Zwischenraum zwischen einem Paar Flansche (74,474) überbrückt, welche von dem distalen Teil radial nach außen abstehen.

33. Halter nach Anspruch 32, bei dem der distale Teil (26,326,426) eine von dem distalen Teil abstehende Einfassung (430A) aufweist, die den Überbrückungsteil (429C) aufweist und die Einrichtung zum Steuern der Zuführung der Substanz abdeckt.

34. Halter nach Anspruch 22, bei dem das erste Ende des Arzneimittelbehälters eine Sprühdüse (50,650) zur Verwendung bei der intranasalen Zuführung der Substanz aufweist und der Arzneimittelbehälter eine Spritze ist.

35. Halter nach Anspruch 34, ferner mit einem mit einem ersten Ende des distalen Teils verbundenen Begrenzungsteil (502), der die Einsetztiefe der Sprühdüse in ein Nasenloch begrenzt.

36. Halter nach Anspruch 35, bei dem der Begrenzungsteil (502) gekrümmt ist, um auf spezifische Bereiche in einer Nasenhöhle zu zielen.

37. Halter nach Anspruch 34, bei dem die Sprühdüse (50,650) gekrümmt ist, um auf spezifische Bereiche in einer Nasenhöhle zu zielen.

38. Halter nach Anspruch 22, ferner mit einer Kappe (702), die mindestens ein erstes Ende des distalen Teils abdeckt.

39. Halter nach Anspruch 38, bei dem die Kappe (702) eine Öffnung (729B) aufweist, die von einem Teil der Kappe gebildet ist, der über einen Zwischenraum zwischen den Flanschen verläuft.

40. Halter nach Anspruch 38, bei dem die Kappe eine Missbrauchserkennungseinrichtung (729A,729B) aufweist.

## Revendications

1. Système (20) pour délivrer au moins une substance en au moins deux doses, comprenant :
un récipient de médicament (22) comprenant un corps cylindrique (30), une première extrémité s'étendant à partir dudit corps cylindrique, et une butée (40) positionnée de manière coulissante à l'intérieur dudit corps cylindrique ;
un support (24, 324, 424) ayant une partie distale (26, 326, 426) et une partie proximale (28, 328, 428), la partie distale étant assemblée à la partie proximale, et le récipient de médicament fixé étant à l'intérieur de celle-ci ; et
des moyens pour contrôler la distribution d'une substance contenue dans le corps cylindrique du récipient de médicament comprenant une pluralité de fentes (90, 92) s'étendant de manière axiale le long d'au moins l'une desdites parties du support, moyennant quoi suite à l'activation dudit système, lesdites parties dudit support se déplacent l'une vers l'autre suite à l'application d'une force minimum et ladite butée (40) se déplace sur une distance axiale présélectionnée pour expulser au moins une partie de ladite substance dudit récipient de médicament,
comprenant :
une paire de nervures extérieures (76) sur ladite partie distale (26, 326, 426) dudit support (24, 324, 424), une paire de rebords (74) fixés auxdites nervures (76), dans lequel lesdites fentes (90, 92) dans ladite partie proximale dudit support comprennent au moins une paire de fentes situées sur celles-ci, ladite paire de fentes comprenant une première fente (90) et une seconde fente (92) s'étendant axialement le long du corps de la partie proximale du support généralement parallèlement entre elles et dimensionnées et situées pour loger les nervures (76) de sorte que l'une desdites nervures peut être insérée dans chaque fente et pouvant se déplacer le long de la fente suite à l'activation du système,
dans lequel ladite partie distale et ladite partie proximale dudit support étant fixées entre elles au moyen d'éléments flexibles (329A, 429A) et des ouvertures correspondantes (329B, 429B) pour éviter l'activation prématurée du système.

2. Système selon la revendication 1, dans lequel lesdites ouvertures (329B, 429B) sont formées par une partie (429C) de ladite partie distale reliant un espace entre une paire de rebords (74, 474) s'étendant radialement vers l'extérieur à partir de ladite partie distale.

3. Système selon la revendication 2, dans lequel ladite partie distale (26, 326, 426) comprend une jupe (430A) s'étendant à partir de la partie distale, comprenant ladite partie de pont (429C) et recouvrant les moyens pour contrôler la distribution de la substance.

4. Système selon la revendication 1, dans lequel ladite première extrémité dudit récipient de médicament comprend une buse de pulvérisation (50, 650) destinée à être utilisée pour l'administration intranasale de la substance et ledit récipient de médicament est une seringue.

5. Système selon la revendication 4, comprenant en outre un limiteur (502) associé à une première extrémité de ladite partie distale, et ledit limiteur limitant la profondeur d'insertion de la buse de pulvérisation dans une narine.

6. Système selon la revendication 5, dans lequel ledit limiteur (502) est formé avec une conception incurvée pour cibler les zones spécifiques dans une cavité nasale.

7. Système selon la revendication 4, dans lequel ladite buse de pulvérisation (50) est formée selon une conception incurvée pour cibler les zones spécifiques dans une cavité nasale.

8. Système selon la revendication 1, comprenant en outre un capuchon (702) recouvrant au moins une première extrémité de ladite partie distale.

9. Système selon la revendication 8, dans lequel ledit capuchon (702) comprend une ouverture (729B) qui est formée par une partie du capuchon s'étendant sur un espace entre les rebords.

10. Système selon la revendication 8, dans lequel ledit capuchon comprend des moyens d'inviolabilité (729A, 729B).

11. Système selon la revendication 1, dans lequel ladite partie distale (26, 326, 426) et ladite partie proximale (28, 328, 428) dudit support (24, 324, 424) ont chacune un intérieur généralement tubulaire configuré pour loger ledit récipient de médicament rempli avec une substance à administrer et ladite partie proximale dudit support comprend une extrémité fermée (82) ayant une tige (84) s'étendant à partir de celle-ci pour la mise en prise avec ladite butée dudit récipient de médicament suite à l'activation.

12. Système selon la revendication 1, dans lequel ladite distance axiale présélectionnée correspond à environ un dosage de la substance maintenue dans ledit corps cylindrique de récipient de médicament que l'on souhaite administrer lors d'un premier mouvement de ladite butée.

13. Système selon la revendication 12, dans lequel ladite distance axiale présélectionnée correspond à environ la moitié de la distance sur laquelle ladite butée est capable de se déplacer à l'intérieur dudit corps cylindrique pour administrer environ la moitié de la substance contenue par ledit récipient de médicament.

14. Système selon la revendication 1, ledit système comprenant en outre des moyens (64, 66) pour fixer ledit récipient de médicament dans ladite partie distale (26, 326, 426) dudit support, ladite partie distale ayant une première extrémité (60) à travers laquelle la première extrémité dudit récipient de médicament peut s'étendre et une seconde extrémité ouverte (62) définissant une ouverture de taille suffisante pour recevoir le récipient de médicament.

15. Système selon la revendication 14, dans lequel ledit récipient de médicament est une seringue ayant un bord s'étendant à partir de son extrémité ouverte et lesdits moyens de fixation de récipient de médicament (64, 66) sont positionnés de manière adjacente à ladite extrémité ouverte (62) de ladite partie distale et comprennent au moins une détente (66) positionnée de manière adjacente à l'extrémité ouverte et dimensionnée de sorte que le bord de la seringue peut être retenu de manière fixe dans ladite partie distale par ladite détente.

16. Système selon la revendication 1, comprenant en outre une paire de rebords (74, 474) s'étendant radialement vers l'extérieur à partir de ladite partie distale dudit support et fixés à celle-ci par une pluralité de nervures (76).

17. Système selon la revendication 1, dans lequel la première fente (90) est de préférence ouverte et est divisée en au moins deux parties (90A, 90B) et positionné de manière adjacente à une extrémité ouverte de la première fente, on trouve un pont (98) s'étendant sur au moins une partie de la fente, le pont étant dimensionné de sorte que lorsque la nervure (76) entre en contact avec le pont et qu'une force suffisante est appliquée contre celle-ci, le pont se fracture pour permettre le passage de la nervure le long de la fente.

18. Système selon la revendication 17, dans lequel une détente (99) est positionnée de manière adjacente à l'extrémité ouverte (80) de la première partie (90A) de la première fente (90) de sorte que la nervure (76) peut être attachée entre la détente et le pont (98) avant l'activation du système, et la seconde partie (90B) de la première fente (90) est au moins légèrement décalée de la première partie (90A) de la première fente (90) et vers la seconde fente (92).

19. Système selon la revendication 18, dans lequel l'autre nervure (76) se déplace le long de la seconde fente (92) pour fournir la stabilité structurelle et la traçabilité, la seconde fente (92) comprenant les moyens de sollicitation (102) pour solliciter la nervure dans la première fente vers la seconde partie de la fente suite au relâchement de la force appliquée par un utilisateur.

20. Système selon la revendication 19, dans lequel lesdits moyens de sollicitation sont adaptés pour comprendre une partie découpée formant un bras déviable (102) ayant une paroi interne (104) associée à la seconde fente (92) de sorte que lorsque les nervures (76) se déplacent le long de leurs fentes respectives, une fente dévie le bras flexible (102) pour amener la partie proximale (28, 328, 428) du support à tourner par rapport à la partie distale (26, 326, 426) autour d'un axe central de sorte que la nervure située dans la première fente (90) peut venir en contact avec un second pont (110) de sorte que suite à l'application d'une force suffisante, le pont (110) se fracture pour permettre le passage de la nervure le long de la seconde partie de la première fente.

21. Système selon la revendication 1, dans lequel ladite partie distale (26, 326, 426) dudit support comprend au moins une fenêtre (78) pour permettre le contrôle visuel du contenu du récipient de médicament situé à l'intérieur du support.

22. Support (24) pour le récipient de médicament selon l'une quelconque des revendications 1 à 21, comprenant :
une partie distale (26, 326, 426) et une partie proximale (28, 328, 428), chacune étant configurée pour loger un récipient de médicament (22) rempli avec une substance à administrer, la partie distale pouvant être assemblée à la partie proximale ; ladite partie distale comprenant une paire de nervures extérieures (76) et une paire de rebords (74) fixée auxdites nervures (76);
des moyens pour contrôler l'administration de la substance comprenant une pluralité de fentes (90, 92) s'étendant de manière axiale le long d'au moins l'une desdites parties du support, et positionnées pour loger les nervures de sorte que l'une desdites nervures peut être insérée dans chaque fente et pouvant se déplacer le long de la fente suite à l'activation du système, moyennant quoi lorsque lesdites parties dudit support sont déplacées l'une vers l'autre sur une distance axiale présélectionnée suite à l'application d'une force minimum, au moins une partie de la substance peut être expulsée dudit récipient de médicament, dans lequel ladite partie distale et ladite partie proximale dudit support étant fixées entre elles au moyen d'éléments flexibles (329A, 429A) et d'ouvertures correspondantes (329B, 429B) pour éviter l'activation prématurée du système.

23. Support selon la revendication 22, dans lequel une première extrémité dudit récipient de médicament comprend une buse de pulvérisation (50) et ledit récipient de médicament est une seringue, et ladite partie distale (26, 326, 426) et ladite partie proximale (28, 328, 428) ont chacune un intérieur généralement tubulaire configuré pour loger ladite seringue et ladite partie proximale dudit support (24) comprend une extrémité fermée (82) ayant une tige (84) s'étendant à partir de celle-ci pour la mise en prise avec ladite butée de ladite seringue pendant l'activation.

24. Support selon la revendication 22, dans lequel ladite distance axiale présélectionnée correspond à environ un dosage de la substance contenue dans ledit récipient de médicament que l'on souhaite administrer.

25. Support selon la revendication 24, dans lequel ladite partie distale (26, 326, 426) comprend en outre des moyens (64, 66) pour fixer ledit récipient de médicament à l'intérieur de celle-ci, ladite partie distale ayant une première extrémité (60) à travers laquelle une première extrémité dudit récipient de médicament peut s'étendre et une seconde extrémité ouverte (62) définissant une ouverture de taille suffisante pour recevoir un corps cylindrique du récipient de médicament.

26. Support selon la revendication 25, comprenant en outre une paire de rebords (74, 474) s'étendant radialement vers l'extérieur à partir de ladite partie distale et fixés le long de celle-ci par les nervures (76).

27. Support selon la revendication 26, dans lequel lesdites fentes (90, 92) dans ladite partie proximale dudit support comprennent au moins une paire de fentes situées sur celle-ci, ladite paire de fentes comprenant une première fente (90) et une seconde fente (92) s'étendant de manière axiale le long du corps de la partie proximale du support généralement parallèlement entre elles et dimensionnées et positionnées pour loger les nervures de sorte que les nervures peuvent être insérées dans les fentes et peuvent se déplacer le long des fentes suite à l'activation du système.

28. Support selon la revendication 27, dans lequel la première fente (90) est de préférence ouverte et est divisée en au moins deux parties (90A, 90B) et positionné de manière adjacente à une extrémité ouverte de la première fente, on trouve un pont (98) s'étendant sur au moins une partie de la fente, le pont étant dimensionné de sorte que lorsqu'une nervure (76) entre en contact avec le pont et qu'une force suffisante est appliquée contre celle-ci, le pont se fracture pour permettre le passage de la nervure le long de la fente.

29. Support selon la revendication 28, dans lequel une détente (99) est positionnée de manière adjacente à ladite extrémité ouverte (80) de la première partie (90A) de la première fente (90) de sorte que la nervure (76) peut être attachée entre la détente et le pont (98) avant l'activation du système, et la seconde partie (90B) de la première fente (90) est au moins légèrement décalée de la première partie (90A) de la première fente (90) et vers la seconde fente (92).

30. Support selon la revendication 29, dans lequel l'une desdites nervures (76) se déplace le long de la seconde fente (92) pour fournir la stabilité structurelle et la traçabilité, la seconde fente comprenant des moyens de sollicitation (102) pour solliciter la nervure (76) dans la première fente (90) vers la seconde partie (90B) de la fente suite au relâchement de la force appliquée par un utilisateur.

31. Support selon la revendication 22, dans lequel ladite partie distale (26, 326, 426) comprend au moins une fenêtre (78) pour permettre le contrôle visuel du contenu du récipient de médicament lorsqu'il est positionné à l'intérieur du support.

32. Support selon la revendication 22, dans lequel lesdites ouvertures (329B, 429B) sont formées par une partie (429C) de ladite partie distale reliant un espace entre une paire de rebords (74, 474) s'étendant radialement vers l'extérieur à partir de ladite partie distale.

33. Support selon la revendication 32, dans lequel ladite partie distale (26, 326, 426) comprend une jupe (430A) s'étendant à partir de la partie distale, comprenant ladite partie de pont (429C) et recouvrant les moyens pour contrôler l'administration de la substance.

34. Support selon la revendication 22, dans lequel ladite première extrémité dudit récipient de médicament comprend une buse de pulvérisation (50, 650) destinée à être utilisée pour l'administration intranasale de la substance et ledit récipient de médicament est une seringue.

35. Support selon la revendication 34, comprenant en outre un limiteur (502) associé à une première extrémité de ladite partie distale, et ledit limiteur limitant la profondeur d'insertion de la buse de pulvérisation dans une narine.

36. Support selon la revendication 35, dans lequel ledit limiteur (502) est formé selon une conception incurvée pour cibler les zones spécifiques dans une cavité nasale.

37. Support selon la revendication 34, dans lequel ladite buse de pulvérisation (50, 650) est formée selon une conception incurvée pour cibler les zones spécifiques dans une cavité nasale.

38. Support selon la revendication 22, comprenant en outre un capuchon (702) recouvrant au moins une première extrémité de ladite partie distale.

39. Support selon la revendication 38, dans lequel ledit capuchon (702) comprend une ouverture (729B) qui est formée par une partie du capuchon s'étendant sur un espace entre les rebords.

40. Support selon la revendication 38, dans lequel ledit capuchon comprend des moyens d'inviolabilité (729A, 729B).
